# EUROPEAN PATENT APPLICATION

(11) **EP 2 404 992 A1**
(43) Date of publication of application: **11.01.2012**
(21) Application number: 10748818.1
(22) Date of filing: 04.03.2010
(51) Int. Cl.: C12M 3/04, C12Q 1/02, G01N 33/15, G01N 33/50

(54) **CELL EVALUATION SYSTEM USING CELL SHEET AND METHOD FOR USING SAME**

(30) Priority: 04.03.2009 JP 2009080378
(71) Applicant: Tokyo Women's Medical University, Tokyo 162-8666 (JP)
(72) Inventor: KINOOKA, Masahiro, Suita-shi Osaka 565-0871 (JP); TAKEZAWA, Yasunori, Suita-shi Osaka 565-0871 (JP); TAYA, Masahito, Suita-shi Osaka 565-0871 (JP); SAITO, Atsuhiro, Suita-shi Osaka 565-0871 (JP); SAWA, Yoshiki, Suita-shi Osaka 565-0871 (JP); SHIMIZU, Tatsuya, Tokyo 162-8666 (JP); OKANO, Teruo, Tokyo 162-8666 (JP)
(74) Representative: HOFFMANN EITLE
(86) International application number: PCT/JP2010/053580
(87) International publication number: WO 2010/101225

(57) **Abstract**

Three-dimensional information on a biological parameter relating to at least one selected from viability, proliferating ability, migration, and differentiation of cultured cells can be obtained by a simple two-dimensional analytical technique by constructing a cell evaluation system including a multilayered cell sheet, target cells, and a two-dimensional analyzer.

## Description

### TECHNICAL FIELD

The present invention relates to a cell evaluation system useful in the fields of, for example, drug discovery, pharmaceutics, medicine, and biology and relates to a method for using the system.

### BACKGROUND ART

In recent years, various regenerative medical techniques have received attention for regenerating injured body tissues. For example, in regeneration of myocardial tissue, various studies including the following methods have been developed: regeneration by direct injection of myocardial cells, skeletal myoblast cells, or mesenchymal stem cells into myocardial tissue; and regeneration by transplantation of a myocardial tissue cell sheet obtained with lowered damage by changing the temperature of a specific temperature-responsive substrate on which myocardial tissue cells are cultured (see Patent Document 1), as described below. Clinical studies in human subjects have been already started in some cells. Furthermore, recently, studies for inducing differentiation of embryonic stem cells (ES cells) or mesenchymal stem cells into myocardial cells have been developed, and also studies for differentiating induced pluripotent stem cells (iPS cells) into myocardial cells have been started. Separately, studies for constructing myocardial tissue in vitro by tissue engineering and returning the cells to the body have been performed. It has been already reported that a myocardial tissue-like structure was constructed by culturing neonatal rat myocardial cells using a three-dimensional support of collagen, polylactic acid, or gelatin (see Non-patent Documents 1, 2, and 3). Unfortunately, it has been revealed that there are some obstacles for construction of myocardial tissue having a contraction and relaxation function for supplying blood to the whole body.

Development in cell culture technique is indispensable for developing regenerative medical techniques. Animal cell culture techniques have further progressed, and research and development based on animal cells have been widely conducted in various fields. In the beginning of the development of animal cells as a subject, the cells themselves or the products of the cells were made into products, but it is currently being conducted to design useful medicines by analyzing cells or their surface proteins or to treat patients with cells of the patients by proliferating the cells or enhancing the function of the cells in vitro and then returning the cells to the body. At present, techniques of culturing animal cells is one field on which many researches focus. In particular, the technical field of evaluating medicines and cosmetics using cultured cells is focused as a technique that replaces past experiments using animals, from the viewpoints of homogenization of evaluation conditions and protection of animals.

In such backgrounds, Patent Document 2 and Non-patent Document 5 describe a method of examining a biological parameter of cells embedded in a gel. In a common method of three-dimensionally culturing cells in a tissue-like form in vitro, the state of the cells in a gel cannot be analyzed with a simple apparatus, such as a two-dimensional analyzer, due to the thickness of the gel, and an expensive, large-scale apparatus that allows three-dimensional analysis, such as a confocal microscope, must be always used. In addition, the cells in a gel inevitably have a low cell density, unlike in vivo cells, which are in the state of a high cell density. Such a thin state is not necessarily sufficient as an evaluation system.

Patent Document 3 describes a method using a porous membrane for evaluating cell's function. In methods using porous membranes, usually, cells are evaluated by the degree of permeation into a porous membrane as shown in Patent Document 3, or cells are evaluated by effects on the cells when only substances are allowed to pass through a porous membrane. However, these methods merely observe behaviors of cells against the porous membrane, i.e., an artificial substance, and do not reproduce behaviors of cells in vivo.

Non-patent Document 4 describes an evaluation system using cells on a cell culture substrate. In this technique, one layer of cells cultured on the substrate is used. Unfortunately, provided information is merely of a two-dimensional state, which is absolutely different from the state of tissue in vivo, despite the evaluation system using cells. Thus, it is not necessarily sufficient as a cell evaluation system.

In such backgrounds, Patent Document 4 describes a novel method of culturing cells on a cell culture support, where the surface of the substrate is coated with a macromolecular substance having an upper or lower critical solution temperature of 0 to 80°C in water, the cells are cultured at a temperature not exceeding the upper critical solution temperature or not falling below the lower critical solution temperature, and the cultured cells are detached by increasing or decreasing the temperature of the substrate to exceed the upper critical solution temperature or fall below the lower critical solution temperature, without treatment with an enzyme. Patent Document 5 describes a method of culturing skin cells using this temperature-responsive cell culture substrate at a temperature not exceeding the upper critical solution temperature or not falling below the lower critical solution temperature and then detaching the cultured skin cells with low damage by increasing or decreasing the temperature of the substrate to exceed the upper critical solution temperature or fall below the lower critical solution temperature. Use of the temperature-responsive cell culture substrate has led to a variety of new developments on known culture techniques. The cell sheet obtained thereby can be used in transplant and is anticipated to have a structure resembling a living body, and, thereby, it can be sufficiently expected that the cell sheet can be used as a cell evaluation system useful in the fields of, for example, drug discovery, pharmaceutics, medicine, and biology.

### CITATION LIST

### PATENT DOCUMENT

Patent Document 1: PCT Japanese Translation Patent Republication No. 2002-008387 (WO2002/008387)
Patent Document 2: Japanese Unexamined Patent Application Publication No. 2007-259829
Patent Document 3: Japanese Unexamined Patent Application Publication No. 2008-118900
Patent Document 4: Japanese Unexamined Patent Application Publication No. Hei 2-211865
Patent Document 5: Japanese Unexamined Patent Application Publication No. Hei 5-192138

### NON-PATENT DOCUMENT

Non-patent Document 1: FASEB J., 11,683-694 (1997)
Non-patent Document 2: Am. J. Physiol., 227, H433-H444 (1999)
Non-patent Document 3: J. Thorac. Cardiovasc. Surg., 119, 368-375 (2000)
Non-patent Document 4: Experimental Cell Research, 268, 36-44 (2001)
Non-patent Document 5: Gene Therapy, 8, 523-533 (2001)

### SUMMARY OF INVENTION

### TECHNICAL PROBLEM

The present invention has been accomplished for solving the above-described problems involved in cell evaluation systems. That is, the present invention provides a novel temperature-responsive cell culture substrate based on an idea quite different from the conventional techniques.

### SOLUTION TO PROBLEM

The present inventors have researched and developed by investigation from various angles in order to solve the above-mentioned problems and, as a result, have found that a multilayered cell sheet prepared by using a temperature-responsive culture substrate generates a specific environment resembling an in vivo structure. Furthermore, the present inventors have found that the state of cells in the multilayered cell sheet can be varied by changing the type of cells constituting the multilayered cell sheet to produce various types of pseudo-tissue. The present invention has been completed based on these findings.

That is, the present invention provides a cell evaluation system including a multilayered cell sheet, target cells, and a two-dimensional analyzer to obtain three-dimensional information on a biological parameter relating to at least one selected from viability, proliferating ability, migration, and differentiation of the target cells by a two-dimensional analytical technique. The present invention also provides a method of evaluating cells by obtaining three-dimensional information on a biological parameter relating to at least one selected from viability, proliferating ability, migration, and differentiation of the target cells by a two-dimensional analytical technique using the cell evaluation system. The present invention provides three-dimensional information by continuously analyzing two-dimensional information that can be obtained by a simple technique, without direct measurement of the three-dimensional structure of a multilayered cell sheet using a large-scale apparatus.

### ADVANTAGEOUS EFFECTS OF INVENTION

In the cell evaluation system according to the present invention, refined three-dimensional information on a biological parameter of cultured cells can be obtained by a simple technique, two-dimensional analysis. Known observation of cell behavior in tissue requires a large scale analyzer. In contrast, the present invention does not require such an analyzer, and the system of the invention can be easily combined with any peripheral cell culture apparatus to obtain refined information.

### BRIEF DESCRIPTION OF DRAWINGS

Fig. 1 is a diagram schematically illustrating Example 1.

Fig. 2 is a diagram illustrating a procedure of layering cell sheets in Example 1.

Fig. 3 is a diagram illustrating a procedure of evaluating the behavior of cells in Example 1.

Fig. 4 is a diagram illustrating a procedure of evaluating the behavior of cells in Example 1.

Fig. 5 shows the results of co-culture of endothelial cells in multilayered myoblast sheets in Example 1. specifically, the morphology of endothelial cells in multilayered myoblast sheets at 96 h, wherein (A): 0 layer, (B): 1 layer, (C): 2 layers, (D): 5 layers, green: CD31 positive (endothelial cells), red: Celltracker orange positive (myoblast cells), and scale bar: 200 mm. In the drawings, the portions with light colors correspond to the "green" in the footnote.

Fig. 6 shows the results of variations in behaviors of endothelial cells at different numbers of sheets in EBM-2 culture in Example 1, specifically, the morphology of endothelial cells in multilayered myoblast sheets at 96 h, wherein (A): 0 layer, (B): 1 layer, (C): 5 layers, green: CD31 positive (endothelial cells), red: Celltracker orange positive (myoblast cells), and scale bar: 200 mm. In the drawings, the portions with light colors correspond to the "green" in the footnote.

Fig. 7 shows the results of fluctuation with time in co-culture of a five layer myoblast sheet and endothelial cells in Example 1, specifically, the spatial distribution of endothelial cells in the five sheets of myoblast cells, wherein (A) and (D): 0 h, (B) and (E): 48 h, (C) and (F): 96 h, green: CD31 positive (endothelial cells), red: Celltracker orange positive (myoblast cells), scale bars (A) to (C): 200 mm, and scale bars (D) to (G): 20 mm. In the drawings, the portions with light colors correspond to the "green" in the footnote.

Fig. 8 shows the results of fluctuation with time in fluidity in a multilayered myoblast sheet in Example 1, specifically, the 3-D images and spatial distribution of colored cells in 5-layer sheet of myoblast cells, wherein (A): 0 h, (B): 48 h, (C): 96 h, green: Celltracker green (basal layer sheet), red: Celltracker red (other layer sheet), green line: basal layer sheet, red broken line: other layer sheet, and scale bar: 20 mm. In the drawings, the portions with light colors correspond to the "green" in the footnote.

Fig. 9 shows the results of comparison of migration properties of endothelial cells and sheet fluidity in Example 1, specifically, the spatial distribution of endothelial cells and basal layer sheet in a 5-layer sheet of myoblast cells, wherein (A): 0 h, (B): 48 h, green: CD31 positive (endothelial cells), red: Celltracker orange positive (basal layer sheet), green line: endothelial cells, red broken line: myoblast cells, and scale bar: 20 mm. In the drawings, the portions with light colors correspond to the "green" in the footnote.

Fig. 10 shows the results of influence of EGF on network formation in Example 1, specifically, the morphology of endothelial cells in a 5-layer sheet of myoblast cells, wherein (A) and (C): EGF(-), (B) and (D): EGF(+), (A) and (B): 48 h, (C) and (D): 96 h, green: CD31 positive (endothelial cells), red: Celltracker orange positive (myoblast cells), and scale bar: 200 mm. In the drawings, the portions with light colors correspond to the "green" in the footnote.

Fig. 11 shows the results of influence of EGF on network formation in Example 1, specifically, the quantification of endothelial network (1 image: 0.889 mm²).

Fig. 12 shows the results of influence of EGF on network formation in Example 1, specifically, the spatial distribution of endothelial cells in a 5-layer sheet of myoblast cells, wherein (A) and (C): EGF(-), (B) and (D): EGF(+), (A) and (B): 48 h, (C) and (D): 96 h, green: CD31 positive (endothelial cells), red: Celltracker orange positive (myoblast cells), green line: endothelial cells, red broken line: myoblast cells, and scale bar: 20 mm. In the drawings, the portions with light colors correspond to the "green" in the footnote.

Fig. 13 shows the results of influence of EGF on myoblast sheet fluidity in Example 1. specifically, the 3-D images and spatial distribution of colored cells in a 5-layer sheet of myoblast cells, wherein (A) and (C): EGF(-), (B) and (D): EGF(+), (A) and (B): 48 h, (C) and (D): 96 h, green: Celltracker green (basal layer sheet), red: Celltracker red (other layer sheet), green line: basal layer sheet, red broken line: other layer sheet, and scale bar: 20 mm. In the drawings, the portions with light colors correspond to the "green" in the footnote.

Fig. 14 shows the results of comparison of migration properties of vascular endothelial cells to a multilayered cell sheet composed of only the myoblast sheets in Example 2 and to a multilayered cell sheet containing myoblast cells and fibroblast cells at a mixture ratio of 50/50. In the drawings, the portions with light colors are the vascular endothelial cells.

Fig. 15 shows the culture states of myoblast cells in Example 3, specifically, the photographs of myoblast at 48 h in (a) low- and (b) high-density cultures (scale bar: 100 mm).

Fig. 16 shows influence of culture states of myoblast cells on cytokine production characteristics in Example 3, specifically, the cellular production rates of VEGF at 48 h in low- and high-density cultures, accompanied with that in culture of a multilayered myoblast sheet.

Fig. 17 is a diagram illustrating a procedure of layering cell sheets in Example 4.

Fig. 18 shows the results of co-culture of endothelial cells in myoblast sheets in Example 4. In the drawings, the portions with light colors correspond to the vascular endothelial cells (HUVEC).

Fig. 19 shows the results of co-culture of endothelial cells in a cancer cell sheet in Example 5. In the drawings, the portions with light colors correspond to the vascular endothelial cells (HUVEC).
Fig. 20 shows a culture procedure and the results of co-culture of endothelial cells in a cancer cell sheet in Example 6. In the drawings, the portions with light colors correspond to the vascular endothelial cells (HUVEC).

### DESCRIPTION OF EMBODIMENTS

The present invention relates to a new cell evaluation system including a multilayered cell sheet, target cells, and two-dimensional analyzer, wherein the multilayered cell sheet (hereinafter may be referred to as a culture format or packed cells) functions as a scaffold for culturing the target cells. In the system of the present invention, three-dimensional information on biological parameters relating to at least one selected from viability, proliferating ability, migration, and differentiation of the target cells in the culture scaffold can be obtained by a two-dimensional analytical technique. Any two-dimensional analyzer that can obtain two-dimensional information or plane information can be used without limitation. Examples of the analyzer include microscopes such as a fluorescence microscope, an optical microscope, a stereoscopic microscope, a laser microscope, a confocal microscope, and a confocal laser scanning microscope; plate readers; and other devices having macro lenses. The analysis may be performed by observation of an image or by visual observation under a microscope. The analytical technique may be any usual method using the above-mentioned analyzer and is not particularly limited. A typical example of the technique includes methods in which fluorescence staining and/or dye staining of cells are/is performed by means of at least one of reagents, proteins, genes, and other substances, and the degree of staining is observed with, for example, one of the above-mentioned microscopes. The term "labeled cells" refers to these fluorescence and/or dye stained cells.

In this present invention, a multilayered cell sheet is necessary, and in order to prepare this multilayered cell sheet, a temperature-responsive substrate is necessary. This substrate is a cell culture substrate that has a specific surface and is grafted with a temperature-responsive polymer by electron beam irradiation. Any material may be used for the substrate, without limitation, and a surface to which cells adhere may be made of polystyrene, polycarbonate, polymethyl methacrylate, polyethylene terephthalate, or a combination of thereof. In particular, preferred are polystyrene and polycarbonate, which are usually used as cell culture substrates. The temperature-responsive polymer that is used for coating the substrate has an upper or lower critical solution temperature of 0 to 80°C, more preferably 20 to 50°C, in an aqueous solution. An upper or lower critical solution temperature higher than 80°C may cause undesirable death of cells. An upper or lower critical solution temperature lower than 0°C usually causes a significant reduction in cell growth rate or death of cells and is also undesirable.

A temperature-responsive polymer that is used in the present invention may be a homopolymer or a copolymer. Examples of these polymers are described in, for example, Japanese Unexamined Patent Application Publication No. Hei 2-211865 (JP-A-211865/1990). Specifically, the temperature-responsive polymer can be prepared by, for example, homopolymerization or copolymerization of the following monomers. Examples of the usable monomer include (meth)acrylamide compounds, N-(or N,N-di)alkyl-substituted (meth)acrylamide derivatives, and vinyl ether derivatives. In the case of copolymers, any two or more of these monomers can be used. Furthermore, the monomers may be copolymerized with other monomers, or the resulting polymers may be subjected to graft polymerization or copolymerization. Mixtures of these polymers and copolymers may also be used. The polymers can also be crosslinked to the extent that will not impair their inherent properties. Since cells are cultured and detached, separation is performed in a temperature range of 5 to 50°C. Accordingly, examples of the temperature-responsive polymer include poly-N-n-propylacrylamide (lower critical solution temperature of homopolymer: 21°C), poly-N-n-propylmethacrylamide (lower critical solution temperature of homopolymer: 27°C), poly-N-isopropylacrylamide (lower critical solution temperature of homopolymer: 32°C), poly-N-isopropylmethacrylamide (lower critical solution temperature of homopolymer: 43°C), poly-N-cyclopropylacrylamide (lower critical solution temperature of homopolymer: 45°C), poly-N-ethoxyethylacrylamide (lower critical solution temperature of homopolymer: about 35°C), poly-N-ethoxyethylmethacrylamide (lower critical solution temperature of homopolymer: about 45°C), poly-N-tetrahydrofurfurylacrylamide (lower critical solution temperature of homopolymer: about 28°C), poly-N-tetrahydrofurfurylmethacrylamide (lower critical solution temperature of homopolymer: about 35°C), poly-N,N-ethylmethylacrylamide (lower critical solution temperature of homopolymer: 56°C), and poly-N,N-diethylacrylamide (lower critical solution temperature of homopolymer: 32°C). Nonlimiting examples of the monomer for copolymerization used in the present invention include polyacrylamide, poly-N,N-diethylacrylamide, poly-N,N-dimethylacrylamide, polyethylene oxide, polyacrylic acid and salts thereof, and aqueous polymers such as polyhydroxyethyl methacrylate, polyhydroxyethyl acrylate, polyvinyl alcohol, polyvinyl pyrrolidone, cellulose, and carboxymethyl cellulose.

The amount of a temperature-responsive polymer on the surface of a culture substrate can be in the range of 1.1 to 2.3 µg/cm², preferably 1.4 to 1.9 µg/cm² and more preferably 1.5 to 1.8 µg/cm². A coating amount of 1.1 µg/cm² or less precludes detachment of the cells on the polymer even if a stimulus is applied, which causes a significant reduction in work efficiency and is therefore undesirable. In contrast, a coating amount of 2.3 µg/cm² or more prevents the adhesion of cells to such a region and thus sufficient adhesion. The form of the culture substrate in the present invention is not particularly limited, and examples thereof include dishes, multiplates, flasks, and cell inserts.

In the method of the present invention, the cultured cell sheet can be detached and harvested from the temperature-responsive substrate by adjusting the temperature of the culture substrate to which the cultured cells are adhering to a temperature not lower than the upper critical solution temperature or not higher than the lower critical solution temperature of the coating polymer on the culture substrate. The harvest may be performed in the culture solution or in another isotonic solution, and the solution can be selected depending on the purpose. In order to detach and harvest the cells more quickly and more efficiently, a method of lightly tapping or shaking the substrate and a method of stirring the culture medium with a pipette may be employed alone or in combination. Culturing conditions other than the temperature are not particularly limited and can be those in usual methods. For example, the culture medium that is used may be a known one, e.g., a medium containing serum such as fetal calf serum (FCS) or a serum-free medium in which such a serum is not added.

The substrate that is coated with a polymer may be any substrate that can be generally molded, such as polymer compounds other than the above-mentioned ones and ceramics, as well as compounds that are usually used in cell culturing, such as glass, modified glass, polystyrene, and polymethylmethacrylate.

The culture substrate may be coated with a temperature-responsive polymer by any method without particular limitation, for example, by a method described in Japanese Unexamined Patent Application Publication No. Hei 2-211865 (JP-A-211865/1990). That is, the coating can be performed by subjecting the substrate and the above-mentioned monomer or polymer to, for example, irradiation with electron beams (EB), γ-rays, or ultraviolet rays, plasma treatment, corona treatment, an organic polymerization reaction, or physical adsorption such as coating or kneading.

The multilayered cell sheet that is used in the present invention may be a multilayered cell sheet which consists of monolayer sheets of a single kind of cells or a combination of monolayer sheets of which each is formed by a different kind of cells. Use of two or more different types of cells causes interaction between the different types of cells to advantageously give a multilayered cell sheet showing higher activity. The position, order, and number of layers can be properly selected without restriction by, for example, using a cell sheet derived from a synovial membrane, which has high adhesiveness, depending on tissue that is coated or supplemented by the multilayered cell sheet. The number of layers is preferably ten or less, preferably eight or less, and more preferably four or less. Cartilage cells can originally survive under an environment of insufficient nutrient supply. However, if more than ten cell sheets are layered, unfavorably, oxygen and nutrients are hardly supplied to the cell sheet in the central portion of the multilayered cell sheet. Such a case can be avoided by a method of constructing a vascular network in the multilayered cell sheet. Nonlimiting examples of the method of constructing a vascular network include a method of mixing vascular endothelial cells in the multilayered cell sheet in advance, a method of layering vascular endothelial cell sheets into the multilayered cell sheet, and a method of constructing a vascular network by burying the multilayered cell sheet in the body.

The method of producing the multilayered cell sheet of the present invention is not particularly limited, and the multilayered cell sheet can be obtained by, for example, detaching cultured cells in a sheet-like form and layering the cultured cell sheets optionally using a cultured cell moving jig. In such a case, the temperature of the culture medium is not particularly limited providing that, if the polymer coating the surface of the culture substrate has an upper critical solution temperature, the temperature does not exceed the upper critical solution temperature while if the polymer has a lower critical solution temperature, the temperature does not fall below the lower critical solution temperature. Needless to say, a low temperature range in which cells cannot grow and a high temperature range in which cells cannot survive are inappropriate for culturing. Culturing conditions other than the temperature are not particularly limited and may be those in usual methods. For example, the culture medium that is used may be a known one, e.g., a medium containing serum such as fetal calf serum (FCS) or a serum-free medium in which such a serum is not added. The cultured cell moving jig that can capture the detached cell sheets can be used without limitation, and examples thereof include membranes and plates, such as porous membranes, paper, and rubber, and sponge. In order to facilitate the stratifying procedure, a jig having a grip and provided with a membrane or plate, such as a porous membrane, paper, or rubber, or sponge may be used.

Thus, in the multilayered cell sheet of the present invention, the cultured cell sheets are detached from a cell culture substrate coated with a temperature-responsive polymer optionally using a cultured cell moving jig without being damaged by proteases represented by dispase and trypsin during the culture. Furthermore, the basal membrane-like protein that is formed between the cells and the substrate during the culture is not broken by enzymes, and the desmosome structure between cells is maintained. As a result, the cultured cell sheets have less structural defects and high strength.

The inventors of the present applicaiton have found that the multilayered cell sheet used in the present invention are densely layered and thereby form a specific culture scaffold that has been used in a conventional cell evaluation system. For example, the culture scaffold in the conventional evaluation system is a sparse state due to the presence of substances other than cells, and thereby the depth profile analysis requires a large-scale analyzer. In contrast, in the multilayered cell sheet of the present invention, cells are packed in a high density, and the sheet has a small thickness. In addition, the environment inside the multilayered cell sheet is tissue-like, and information can be read by merely repeating two-dimensional analysis in the thickness direction. In the present invention, the number of laminated cell sheets is three or more, preferably four or more, and more preferably five or more. If the number of layers is two or less, the multilayered cell sheet is not in a tissue like state and is therefore unsuitable as a multilayered cell sheet of the present invention.

The present invention is evaluated by allowing target cells to migrate in the obtained multilayered cell sheet and observing the behaviors of the cells. The kind of target cells may be appropriately determined depending on the evaluation purpose, without limitation. Examples of cells having high migration properties include, but not limited to, myoblast cells, vascular endothelial cells, mesenchymal stem cells, myocardial cells, and epidermal basal membrane cells. Examples of cells having low migration properties include, but not limited to, fibroblast cells and differentiated epidermal keratinocyte cells.

Similarly, advantageous characteristics of the multilayered cell sheet that is used in the present invention are that the fluidity can be varied by changing the kind of cells in the multilayered cell sheet and, thereby, various types of pseudo-tissue can be produced. The cells that are used in such a case are not particularly limited. As described above, examples of cells having high fluidity include myoblast cells, vascular endothelial cells, mesenchymal stem cells, myocardial cells, and epidermal basal membrane cells. Examples of cells having low fluidity include, but not limited to, fibroblast cells and differentiated epidermal keratinocyte cells. Multilayered cell sheets having various degrees of fluidity can be produced by mixing the cells having high fluidity and cells having low fluidity.

It is believed that the fluidity can be a parameter for not only the multilayered cell sheet for the purpose of the present invention but also the cell sheet for the purpose of, for example, transplantation. For example, as described in examples below, a multilayered cell sheet having higher fluidity more easily allows blood vessels to invade from the body side, resulting in higher compatibility of the transplanted multilayered cell sheet to the body. It is expected that the multilayered cell sheet having high fluidity allows other cells to easily invade therein.

The biological parameter in the present invention is not particularly limited as long as it relates to evaluation of cells, and examples thereof include viability, proliferating ability, migration, and differentiation of cells and combinations of two or more thereof. In the present invention, the parameter may be three-dimensional information on cells. Examples thereof include, but not limited to, tracks on which cells moved, cell network obtained as the result thereof, and the degree of construction of tubular tissue. According to the present invention, refined information, i.e., a biological parameter of cells, can be obtained by a simple evaluation system.

In the present invention, a drug may be applied to the above-mentioned system. The effect of a drug on cells can be revealed by applying the drug to the system of the present invention and observing the behaviors of the target cells. The drug that is used in such a case is not particularly limited and may be appropriately selected depending on the system to be evaluated. Examples of the drug include cytokines such as EGF, HGF, and VEGF; anticancer agents; and differentiation-inducing agents. The present invention can also be expected to be significantly useful as a system for drug discovery and evaluation of drug efficacy.

In the present invention, various evaluation systems can be constructed by changing the combination of a multilayered cell sheet, labeled cells, and a two-dimensional analyzer. In a specific example of the system, but not limited to, a vascular network-constructing mechanism can be evaluated by tracing the migration properties of fluorescence-labeled vascular endothelial cells in a multilayered cell sheet constituted by myoblast cells with a fluorescence microscope. Alternatively, the drug efficacy of an anticancer agent can be evaluated by constituting the multilayered cell sheet by cancer cells and using fluorescence-labeled vascular endothelial cells as the labeled cells and the anticancer agent as the agent. Furthermore, a stem cell differentiation-programming mechanism can be analyzed by constituting the multilayered cell sheet by differentiated cells and using fluorescence-labeled stem cells as the labeled cells and a differentiation-inducing agent as the agent.

The cells of the present invention are not limited, and examples thereof include animal, insect, and plant cells and bacteria. In particular, many animal cells are commercially available, and the use of animal cells is therefore convenient. Examples of the animal from which cells are derived include, but not limited to, human, monkey, dog, cat, rabbit, rat, nude mouse, mouse, guinea pig, hog, sheep, Chinese hamster, cattle, marmoset, and African green monkey.

The cell evaluation system according to the present invention can provide three-dimensional information on a biological parameter relating to at least one selected from viability, proliferating ability, migration, and differentiation of cultured cells by a simple two-dimensional analytical technique. The present invention does not need a large-scale analyzer that has been necessary for observing the behaviors of cells in tissue.

### EXAMPLES

The present invention will be described in further detail with reference to examples below, but should not be limited thereto.

### EXAMPLE 1

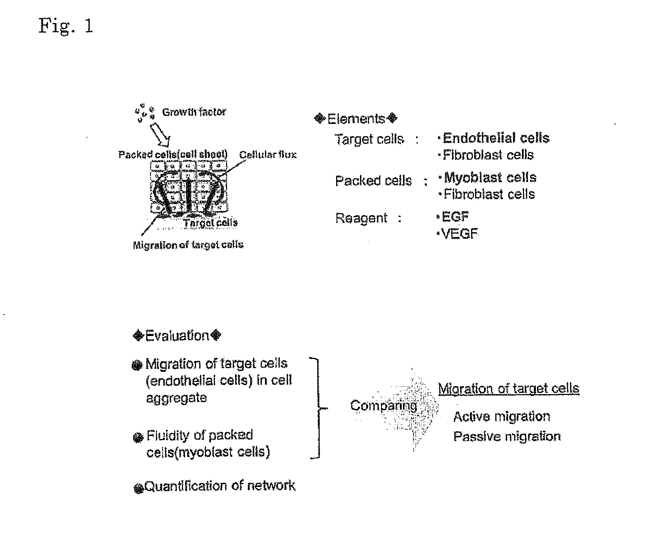

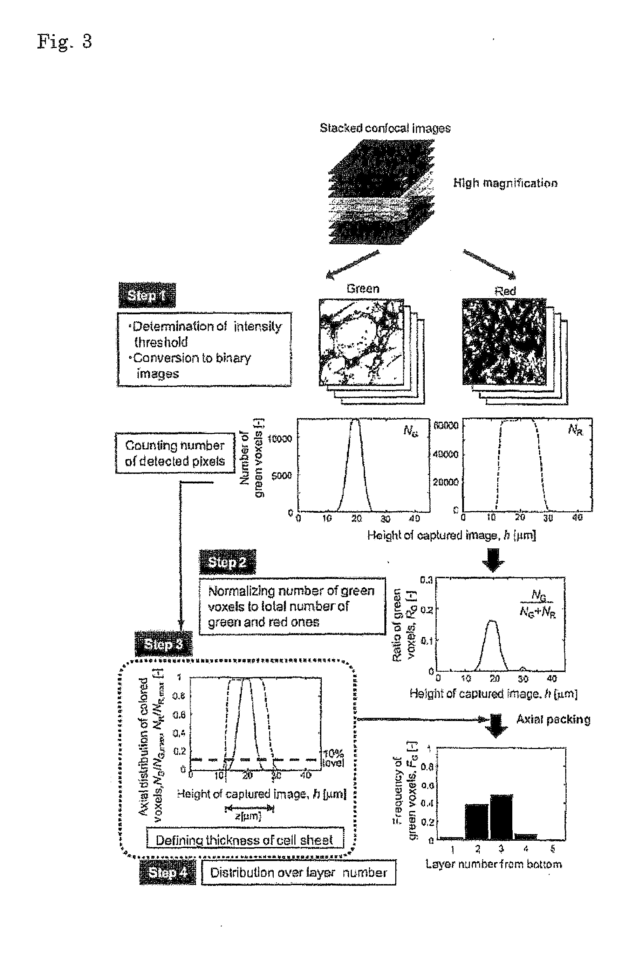

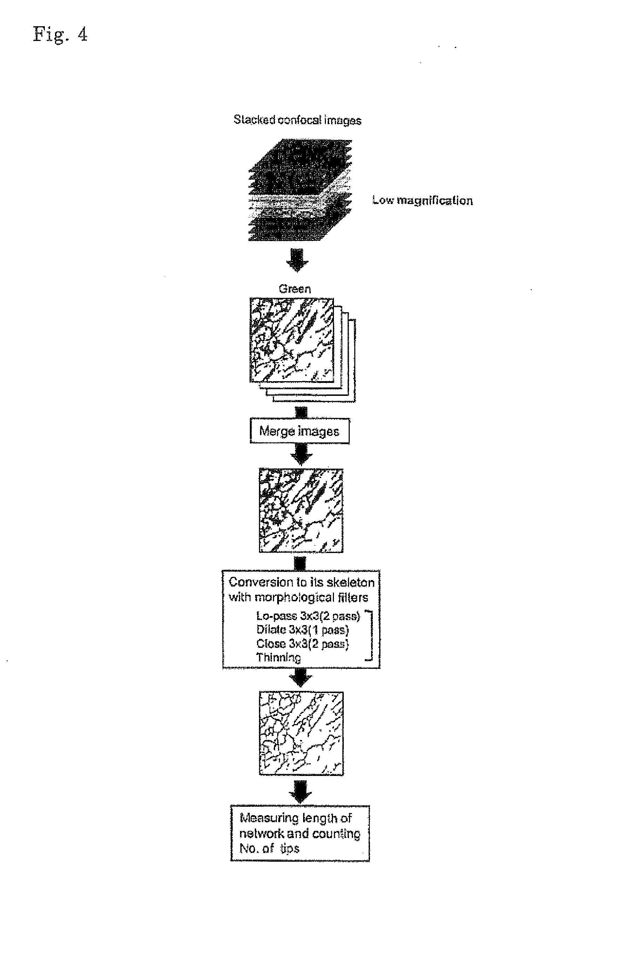

A co-culture system of vascular endothelial cells and a multilayered myoblast sheet was constructed (Fig. 1). This system is composed of three elements: "target cells", "packed cells", and "a drug", wherein the target cells are labeled endothelial cells that migrate in the packed cells; the packed cells are myoblast cells constituting a multilayered cell sheet serving as a scaffold of the labeled cells; and the migration of the labeled cells and the behaviors of the packed cells can be controlled by the stimulus of addition of the drug. Previous studies have already revealed that the cells constituting a multilayered cell sheet migrate in the dense environment of the sheet, and this is called "fluidity" of the packed cells. That is, the labeled cells migrate in a dynamic scaffold. In other words, this system resembles an in vivo environment that is affected by intercellular communications with surrounding other cells and enables analysis under pseudo in vivo conditions in another system by varying the three elements. Then, a method of evaluating the migration of the labeled endothelial cells and the sheet fluidity of the packed cells was established. According to the procedure of evaluation shown in Fig. 3, the migration of the endothelial cells and the sheet fluidity were compared for investigating whether the migration of the endothelial cells is faster than the fluidity of the sheet (active migration) or is passive against the sheet fluidity (passive migration). The quantification of the network was evaluated by the procedure shown in Fig. 4. Furthermore, the effect of an epidermal growth factor (EGF), which is a growth factor of epithelial cells, was investigated by adding EGF as the drug.

### Primary cell culture

Cell culture was conducted using a polystyrene culture vessel (225 cm², T-flask) manufactured by Corning Inc. In the culture of myoblast cells, laminin (manufactured by Sigma) was applied to the culture surface. The laminin-coated surface was produced by applying 1 mL of a solution of laminin diluted 20-fold with a phosphate buffer solution (PBS, manufactured by Sigma) to each 25 cm² of the culture surface, absorbing the protein to the surface by incubation at 37°C for 1 hr in the presence of 5% CO₂, and then washing the surface with PBS.

### Monolayer subculture

In the experiment, scaffold-dependent human skeletal myoblast cells (manufactured by Camblex) and normal human umbilical vein endothelial cells (manufactured by Lonza) were cultured in a CO₂ incubator (MCO-17AI manufactured by Sanyo Electric Co., Ltd.) at 37°C under a 5% CO₂ atmosphere. The myoblast cells were cultured in a Dulbecco's modified Eagles Medium (DMEM, manufactured by Sigma) containing 1 vol% of an antibiotic/antifungal agent 100 x (manufactured by Invitrogen), 2 vol% of a 1 M HEPES buffer (manufactured by Sigma), and 10 vol% of fetal bovine serum (hereinafter referred to as FBS, manufactured by GIBCO) (hereinafter referred to as DMEM (0% FBS)). In the primary and subculture, 1 mL of a trypsin solution containing 0.1 % of trypsin (manufactured by Sigma) and 0.02% of ethylenediamine tetra-acetic acid (EDTA, manufactured by Sigma) was added dropwise onto each 1 cm² of the culture area, followed by a reaction at 37°C for 3 min to detach the cells. A trypsin inhibitor solution (Wako Pure Chemical Industries, Osaka, Japan) in the same amount as that of trypsin was added to the suspension of the cells to terminate the enzyme-distributed reaction. The cells were collected by centrifugation at 1000 rpm at room temperature for 5 min and were then resuspended in the medium. The resulting cell suspension was applied to a culture surface to inoculate the cells at a living cell concentration of 1.0 × 10³ cells/cm², and the medium was supplemented into a depth of 2 mm. The medium was replaced with new one every 24 hr.

The endothelial cells were cultured in EGM-2 Bullet Kit (manufactured by Lonza) (hereinafter referred to as EBM-2). In the primary and subculture, 1 mL of a trypsin solution containing 0.1% of trypsin (manufactured by Sigma) and 0.02% of ethylenediamine tetra-acetic acid (EDTA, manufactured by Sigma) was added dropwise onto each 1 cm² of the culture area, followed by a reaction at 37°C for 3 min to detach the cells. A trypsin inhibitor solution (Wako Pure Chemical Industries, Osaka, Japan) in the same amount as that of trypsin was added to the suspension of the cells to terminate the enzyme-distributed reaction. The cells were collected by centrifugation at 1450 rpm at room temperature for 5 min and were then resuspended in the medium. The resulting cell suspension was applied to a culture surface to inoculate the cells at a living cell concentration of 2.5 × 10³ cells/cm², and the medium was supplemented into a depth of 2 mm. The medium was replaced with new one every 48 hr.

### Co-culture of multilayered myoblast sheet and endothelial cells

A 35 mm polystyrene culture dish (manufactured by Corning) and a temperature-responsive culture vessel (24-well multiwell, manufactured by CellSeed) were used as cell culture substrates. The temperature-responsive culture vessel has a culture surface made of graft polymerization of N-isopropylacrylamide to a polystyrene surface and has a critical point of 32°C, at which the hydrophobicity and hydrophilicity of the surface are reversibly changed. Accordingly, the cells can be easily detached from the culture surface by changing the temperature, while maintaining the intercellular adhesion.

In production of a myoblast sheet, 2.3 × 10⁵ cells/cm² of myoblast cells were seeded in a temperature-responsive culture vessel and were preincubated at 37°C for 24 hr so as to be confluent. After 24 hr, the cells were incubated at 20°C for 30 min under a 5% CO₂ atmosphere, and the detached cell sheet was harvested with a gelatin gel from the upper side. This is the first layer of a cell sheet, and a multilayer cell sheet was produced by repeating the detachment and harvesting of cells.

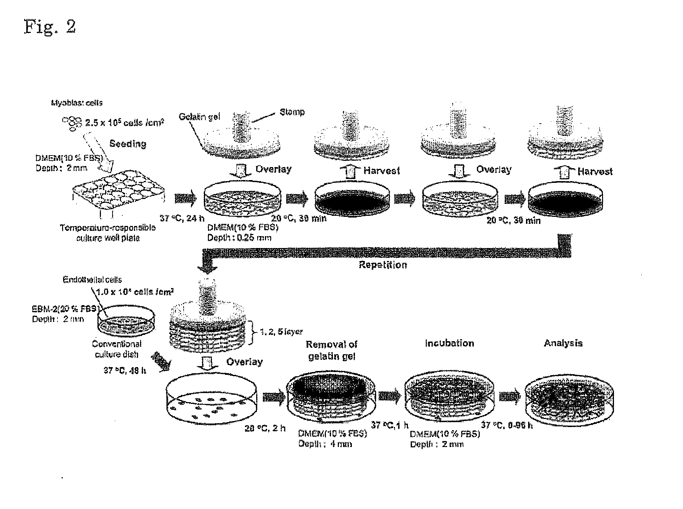

In construction of a sheet co-culture system, 1.0×10⁴ cells/cm² of endothelial cells were seeded in a 35 mm culture dish. EBM-2 (2% FBS) was used as the medium and was replaced with an EBM-2 medium while the concentration of FBS was increased to 20%, which was the optimum concentration for growth of endothelial cells, after 24 hr in order to improve the adhesiveness with the myoblast sheet that was to be overlaid. After 24 hr, the medium was removed, and the myoblast sheet was overlaid later. The number of layers in the multilayered myoblast sheet was variously changed. The gelatin gel was molten at 37°C and removed, and co-culture was started from a time, 0 hr, when the gelatin gel was molten (Fig. 2).

Cytoplasmic staining of the myoblast cells was performed before producing myoblast sheets for detecting the height of the structure and for color coding the sheets in investigation of fluidity of the myoblast cells. One milliliter of a trypsin solution containing 0.1% of trypsin (manufactured by Sigma) and 0.02% of ethylenediamine tetra-acetic acid (EDTA, manufactured by Sigma) was added dropwise onto each 1 cm² of the culture area, followed by a reaction at 37°C for 3 min to detach the cells. A trypsin inhibitor solution (Wako Pure Chemical Industries, Osaka, Japan) in the same amount as that of trypsin was added to the suspension of the cells to terminate the enzyme-distributed reaction. The cells were collected by centrifugation at 1000 rpm at room temperature for 5 min and were then suspended in a serum-free medium. To the resulting cell suspension, the same amount of serum-free medium-diluted 10 µM Celltracker Orange CMTMR (manufactured by Molecular probes) (or Celltracker green CMFDA (manufactured by Molecular probes)) was added such that the concentration of the Celltracker dye was 5 mM. The suspension was left at 37°C for 15 min under a 5% CO₂ atmosphere, and then the cells were collected by centrifugation at 1000 rpm at room temperature for 5 min, resuspended in DMEM containing 10% FBS, and seeded at a concentration of 2.3 × 10⁵ cells/cm².

In order to identify and observe endothelial cells in the structure where both the myoblast cells and the endothelial cells were present, the endothelial cells were stained using an antibody, Anti-CD31 (mouse monoclonal anti-human CD31, manufactured by DAKO). CD31 is an antibody that recognizes a glycoprotein having a molecular weight of 100 kD present in endothelial cells. The cells were washed with FBS and were added to a 4% paraformaldehyde/phosphate buffer solution (manufactured by Wako Pure Chemical Industries), followed by fixation at 4°C overnight. The culture surface was washed with PBS twice, and then 0.1% Triton X-100 diluted with PBS was added thereto to impart permeability to the cells. The culture surface washed with PBS twice and was immersed in 0.1% bovine serum albumin (manufactured by Wako Pure Chemical Industries) diluted with PBS for 1 hr. Subsequently, the culture surface was immersed in anti-CD31 antibody diluted 40-fold with 0.1% bovine serum albumin and was left at 4°C overnight. The culture surface was washed with PBS twice and was immersed in a secondary antibody (Alexa FluorR 488 goat anti-mouse IgG, manufactured by Molecular Probes) diluted 200-fold with 0.1% bovine serum albumin at room temperature for 1 hr. The culture surface was washed with PBS twice. Then, one or two drops of Slow fade (manufactured by Molecular Probes) were added to the culture surface, and a cover glass (manufactured by IWAKI) was put thereon. A three-dimensional image was obtained with a confocal microscope (EX-20, manufactured by OLYMPUS) to observe the cells.

Regarding the fluidity in a multilayered cell sheet, the lowermost layer of the multilayered cell sheet was stained green with Celltracker green CMFDA, and other four layers were stained red with Celltracker orange CMTMR by the above-described method for cytoplasmic staining, and a multilayered myoblast sheet was produced by the above-described procedure (construction of sheet co-culture system). The resulting multilayered cell sheet was placed on a 35 mm culture dish coated with FBS for sheet culture.

### Procedure for evaluation of behaviors of cells

The frequency distribution in the vertical direction was determined from a three-dimensional image obtained with a confocal laser scanning microscope (Fig. 3). First, sliced images of individual colors were converted to binary images using a visually determined threshold (Step 1), and the distribution (R_{G}[-]) in the height direction was determined by normalizing the number of green pixels (N_{G}[voxel]) in one image by the sum of the number of green pixels and the number of red pixels (N_{G}+N_{R}[voxel]) (Step 2). Then, the height (z[µm]) was calculated by defining the portion where the number of pixels was 10% or more of the distribution normalized by the maximum number of pixels (N_{G},max or N_{R},max[voxel]) as a portion of existing stained tissue (Step 3) in each color. The distribution in Step 2 was further normalized within the height range determined in Step 3, and the distribution of green voxels was determined as a frequency distribution (F_{G}[-]) in each layer of the multilayered cell sheet, and the distributions of the endothelial cells and the sheet cells in the lowermost layer of the multilayered cell sheet were determined (Step 4).

In the quantitative evaluation of a vascular network, photographs of the networks taken with a confocal laser scanning microscope were subjected to two-dimensional image analysis of the network using Image ProPlus (Fig. 4). The photographs were each converted to a binary image by a visually determined threshold, and the length of the network and the number of tips of a skeletal image converted with morphological filters were determined to compare individual networks.
Co-culture of endothelial cells in a multilayered myoblast sheet
Variation in behaviors of endothelial cells in a multilayered myoblast sheet having various numbers of sheets

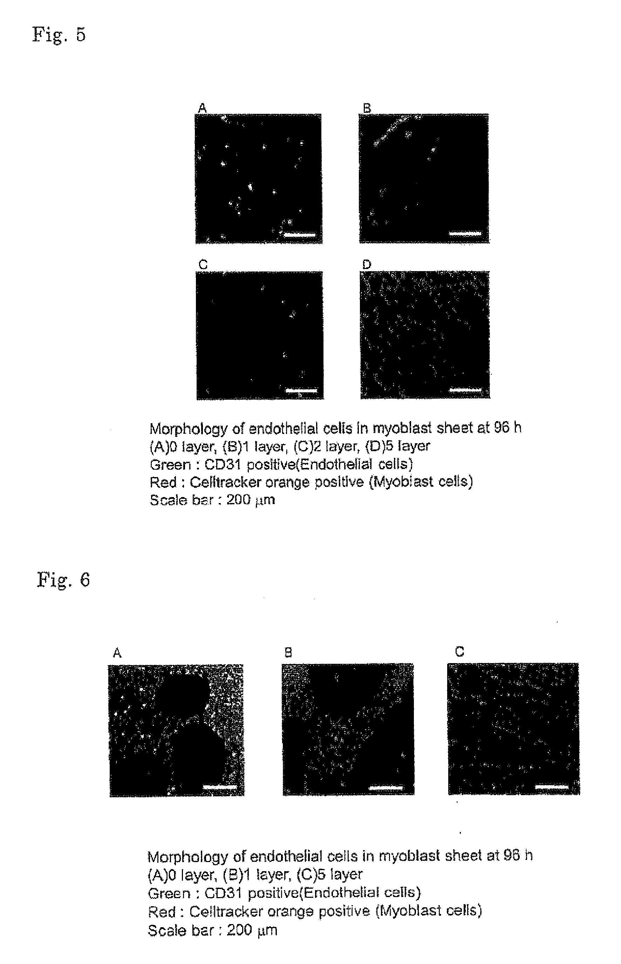

Endothelial cells were cultured in an EBM-2 medium for 48 hr and were then co-cultured with myoblast cells in DMEM (10% FBS) for 96 hr by seeding 2.0 × 10⁴ cells/cm² of myoblast cells stained with Celltracker orange (defined as a zero layer sheet) or a one-, two-, or five-layer myoblast sheet on the endothelial cells. Then, CD31 staining was performed. In the case of the zero layer, endothelial cells were hardly observed, and no network was present (Fig. 5(A)). In the drawings, the portions with light colors correspond to the "green" in the footnote. In monoculture of endothelial cells, detachment of the endothelial cells was already observed about 48 hr after replacement of EBM-2 by DMEM (data not shown), probably due to the detachment of the most of the endothelial cells at 96 hr. In the case of the one- or two-layer myoblast sheet, like the case of the zero layer, the portions where endothelial cells were present were small, and no formation of network was observed (Figs. 5(B) and 5(C)), also probably due to detachment of the cells from the culture surface to desorb through the cells of the sheets. In the case of the five-layer myoblast sheet overlaid on the endothelial cells, a network was formed in a broad range (Fig. 5(D)). The portions where the endothelial cells were present increased compared to the cases of the zero layer and the one- or two-layer sheet. This suggests that since the permeation of the endothelial cells through the sheets becomes difficult with an increase in number of sheets and, thereby, the cells tend to stay in the sheets, the five-layer sheet makes the endothelial cells stay to form a network.

### Variation in behaviors of endothelial cells in EBM-2 culture at various numbers of layers

Fig. 6 shows the results of co-culture of endothelial cells and zero-, one- or five-layer myoblast sheet for 96 hr in EBM-2. In the co-culture with the zero-, or one-layer sheet in DMEM, endothelial cells were hardly present (Figs. 6(A) and 6(B)). In the drawings, the portions with light colors correspond to the "green" in the footnote. Both the endothelial cells and the myoblast cells can adhere to EBM-2, and formed clusters without forming a network. In contrast, in the co-culture with the five-layer sheet, network was formed over a broad range (Fig. 6(C)). These results show that the formation of a network was promoted by the growth in a three-dimensional structure provided by the five-layer sheet, regardless of the type of medium. The network formed in EBM-2 was finer than that formed in DMEM (10% FBS) and was smooth thread like without unevenness. This also shows that the formation of a network was promoted by the growth in a three-dimensional structure provided by the five-layer sheet, regardless of the kind of medium and the adhesiveness to the culture surface. Thus, it was suggested that the multilayered cell sheet functioned as a matrix for endothelial cells and has an ability of forming a network as in in vitro matrices such as matrigel and collagen gel. The multilayered cell sheet, which forms a matrix, probably contributes to establishment of a network-forming system for providing an environment resembling in vivo where endothelial cells grow with being surrounded by other cells. The results suggested that a five-layer sheet was effective for retaining the endothelial cells inside the structure, and DMEM (10% FBS) can be used as the medium. Accordingly, these are defined as fundamental conditions for a network-forming co-culture system.

### Fluctuation with time in co-culture of a five-layer myoblast sheet and endothelial cells

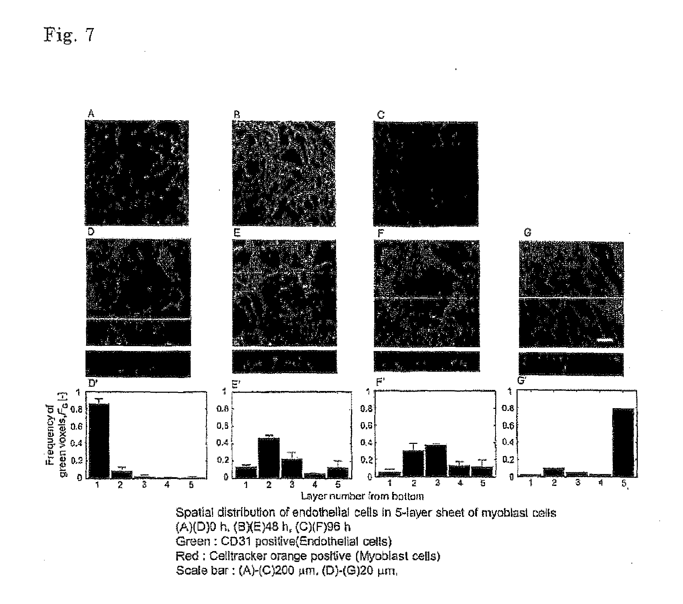

Fig. 7 are photographs illustrating the results of culture of a five-layer myoblast sheet and endothelial cells in DMEM (10% FBS) for 0, 48, or 96 hr, taken confocally. In the drawings, the portions with light colors correspond to the "green" in the footnote. It is obvious from Fig. 7(A) that endothelial cells have already started to bind to one another at 0 hr, i.e., immediately after the melting of the gelatin gel. Since the time of 0 hr is 3 hr after the overlaying of the multilayered myoblast sheet on the endothelial cells due to the procedure of sheet layering, the binding probably has started within this 3 hr. The endothelial cells formed a fibrous network at 48 hr and further formed, at 96 hr, a network having a form that is different from that of the fibrous network formed at 48 hr (Figs. 7(B) and 7(C)). The cross-sectional photograph shows that the endothelial cells are present under the multilayered myoblast sheet at a sheet culture period of 0 hr (Fig. 7(D)). But, at culture periods of 48 and 96 hr, many endothelial cells are present inside the multilayered sheet. Accordingly, it is believed that the endothelial cells migrate upward inside the multilayered sheet (Figs. 7(E) and 7(F)). As shown in Fig. 7(G), clusters were formed on the upper surface of the multilayered sheet at 48 and 96 hr. The vertical distribution demonstrates that the endothelial cells are mostly present in the first layer of the multilayered sheet at 0 hr and migrate upward with the culturing time (Figs. 7(D'), 7(E'), 7(F'), and 7(G')). This suggests that the endothelial cells under the multilayered sheet bind to one another and migrate to the upper portion of the multilayered sheet with passage of time to form a three-dimensional network, and that the cells reach the top of the multilayered sheet form clusters.

### Fluidity in sheet

### Fluctuation in fluidity with time in myoblast sheet

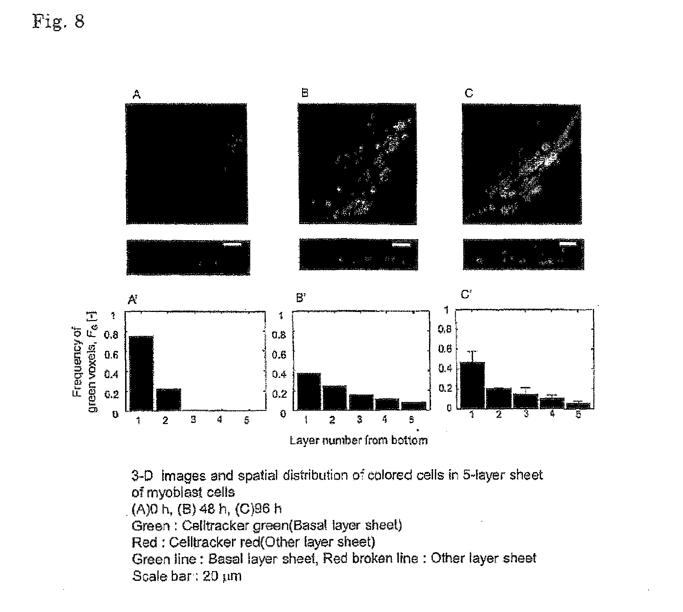

In order to investigate the fluidity of the sheet as an environment and a scaffold for endothelial cells, the lowermost layer of the multilayered sheet was stained with Celltracker green (green) and other four layers were stained with Celltracker orange (red), and these sheets were layered and subjected to sheet culture. Fig. 8 shows three-dimensional images taken confocally, cross-sectional views, and distributions of sheet cells in the lowermost layer. In the drawings, the portions with light colors correspond to the "green" in the footnote. At a time of 0 hr at which the gelatin gel was molten, the lowermost layer and other layers were clearly separated from each other, and most of the cells in the lowermost layer did not migrate upward to maintain the structure (Fig. 8(A)). The photographs after 48 and 96 hr show that the cells in the lowermost layer migrated upward (Figs. 8(B) and 8(C)). The graphs of the distribution in the vertical direction also show that the cells stained green were mostly present in first layer at 0 hr and migrated upward at 48 and 96 hr. This suggests that the cells flow in the dense environment of the sheet.

### Comparison of migration properties of endothelial cells and sheet fluidity

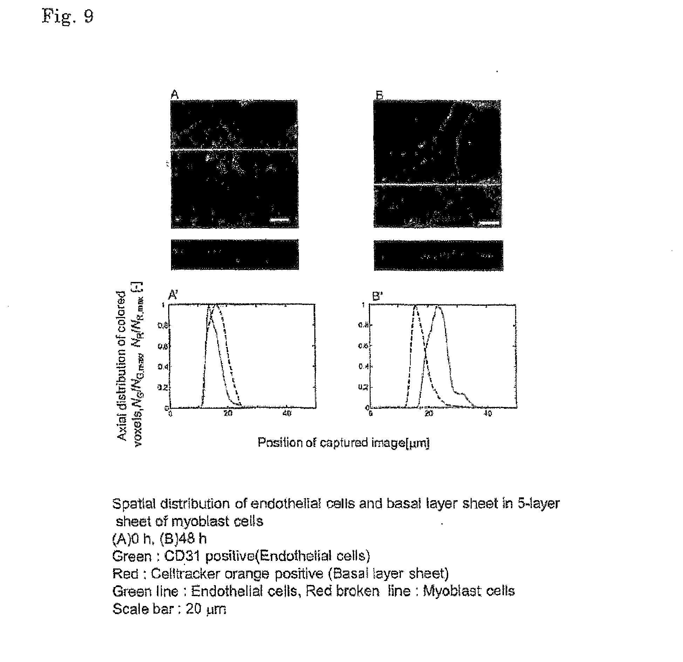

The former section reveals that endothelial cells migrate in a multilayered myoblast sheet and form a network. The migration of the endothelial cells in the vertical direction was compared with the fluidity of the myoblast cells for investigating whether the migration of the endothelial cells is faster than the fluidity of the sheet (active migration) or is passive against the sheet fluidity (passive migration). The distribution of the endothelial cells was compared with the distribution of the sheet cells present in the lowermost layer that was present approximately the same position as the endothelial cells in the vertical direction. The position of the endothelial cells was slightly lower than that of the sheet cells at 0 hr, and the endothelial cells migrated upward at 48 hr (Figs. 9(A) and 9(B), where the portions with light colors correspond to the "green" in the footnote). Accordingly, the endothelial cells migrate faster than the fluidity of the sheet and bind to one another to form a network and clusters. In conclusion, the migration of the endothelial cells is more active than that of the myoblast cells, i.e., active migration.

### Influence of EGF addition

### Influence of EGF on formation of network

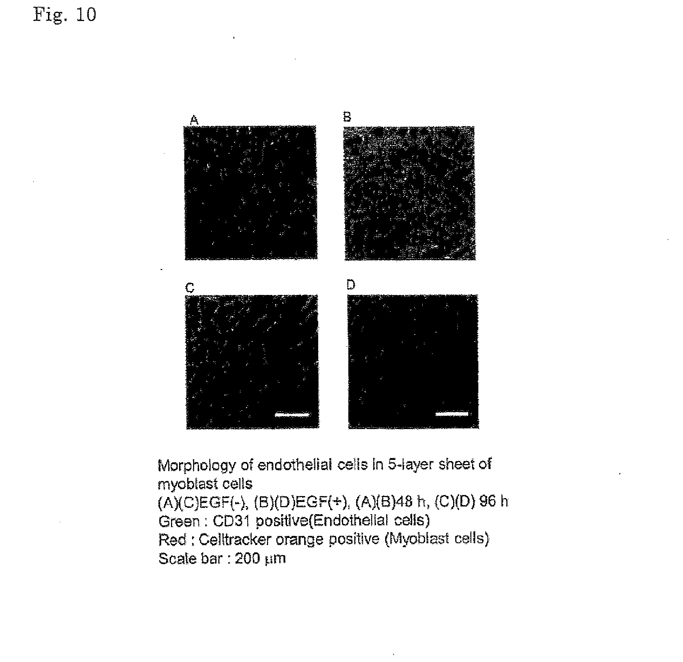

In order to investigate the influence of a drug, EGF (100 ng/mL) was added to DMEM. Fine networks were formed in both DMEM containing EGF and DMEM not containing EGF at 48 hr, and no difference in morphology was observed (Figs. 10(A) and 10(B), where the portions with light colors correspond to the "green" in the footnote). However, at 96 hr, the network formed in the DMEM containing EGF was finer than that in the DMEM not containing EGF (Figs. 10(C) and 10(D)).

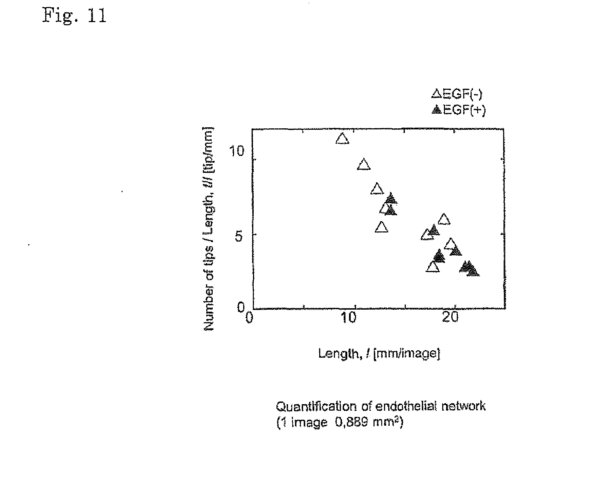

The endothelial cells formed a three-dimensional network in the five-layer sheet. Thus, the endothelial cells formed a network inside the multilayered sheet, i.e., within an environment having a limited height. This suggests that the height can be neglected for two-dimensional quantitative evaluation. Fig. 11 is a graph plotting the network length L (µm/image) and the number of tips T[tip] of skeletal images at 96 hr after image processing. A longer network and a smaller number of tips were observed in the DMEM containing EGF. Thus, these parameters showed that the addition of EGF enhance the binding of the endothelial cells to one another and formation of a fine network. Regarding the image at 48 hr, the skeletal image converted by the same image processing as in that at 96 hr largely differs from that of the initial network and was not able to be quantitatively evaluated.

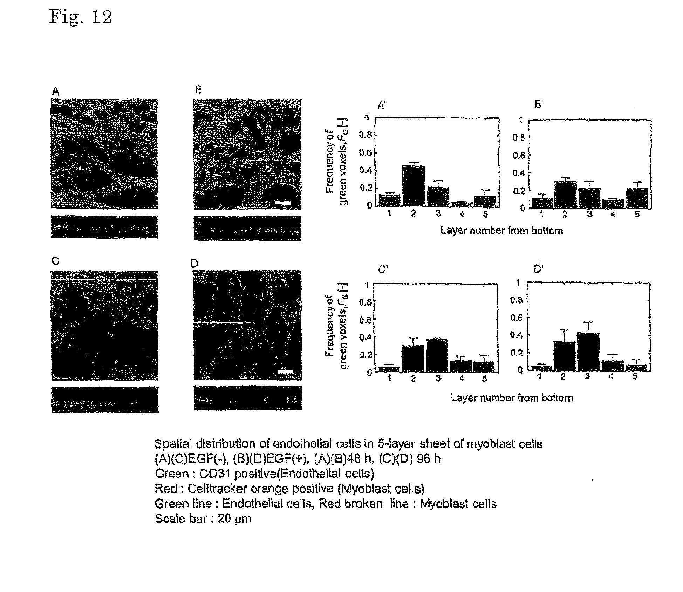

Fig. 12 shows the cross-section photographs and the distribution in the vertical direction of the endothelial cells. In the drawings, the portions with light colors correspond to the "green" in the footnote. Figs. 12(A'), 12(B'), 12(C'), and 12(D') show that there was no difference between the presence and the absence of EGF at 48 and 96 hr and thus the addition of EGF does not affect the migration of endothelial cells. It is believed that the migration rates of the endothelial cells are at approximately the same level under the both conditions and that the frequencies of contact (collision) among the endothelial cells are at approximately the same level under the both conditions. After the contact, some of the cells separate from each other while some of the cells bind to each other. The addition of EGF probably increases the frequency of the binding and, as a result, enhances formation of a finer network.

### Influence of EGF on fluidity of myoblast sheet

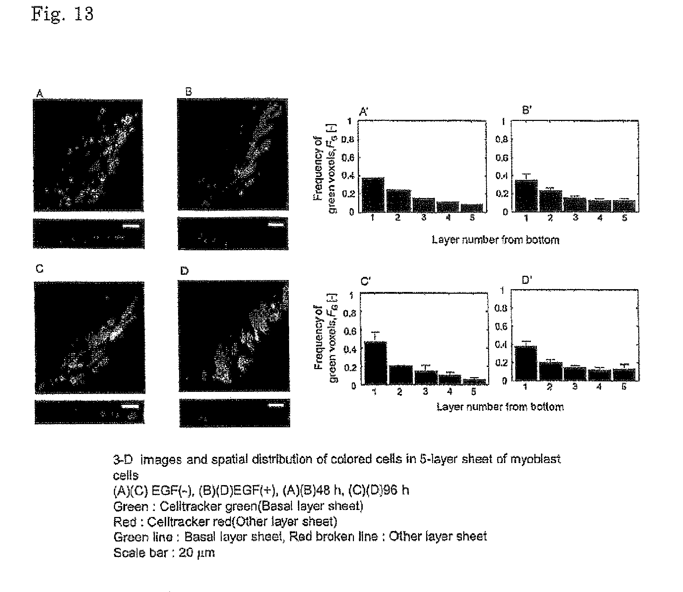

Whether the addition of EGF affect the fluidity of a sheet was investigated. It has been confirmed that the migration of the myoblast cells is enhanced by addition of EGF in monolayer culture. Accordingly, it is supposed that the migration is enhanced also in the sheet. Fig. 13 shows three-dimensional images taken confocally, cross-sectional views, and distributions of sheet cells in the lowermost layer after EGF was added. In the drawings, the portions with light colors correspond to the "green" in the footnote. No influence of the addition of EGF on distribution in the vertical direction was observed at 48 and 96 hr (Figs. 13(A'), 13(B'), 13(C'), and 13(D')). Accordingly, the influence of EGF on the fluidity of the sheet is small, and the enhancement of network formation of the endothelial cells was probably not caused by an increase in fluidity of the sheet. This investigation suggests that the developed network formation is not caused by EGFR activated by the addition of EGF. EGF did not influence both the sheet fluidity and the migration of the endothelial cells in the vertical direction. However, as shown in Fig. 11, the plots in the case of adding EGF lie in the extension of the plots in the case of not adding EGF. Accordingly, the addition of EGF accelerates the adhesion between the cells. This is probably caused by the influence of EGF on adhesion molecules of the endothelial cells through any pathway. In the pathway, if EGF affects the secretion of cytokine by the myoblast cells to increase the expression of bFGF, the bFGF increases the expression of VE-cadherin, an adhesion molecule of the endothelial cell. As a result, adhesiveness between the cells is enhanced to form a fine network.

### EXAMPLE 2

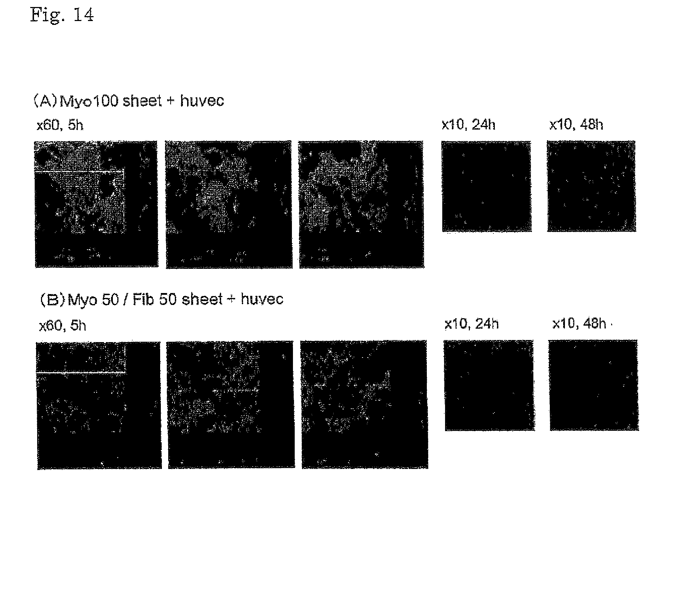

A five-layer myoblast sheet (Fig. 14(A)) composed of myoblast sheets only and a five-layer cell sheet (Fig. 14(B)) including myoblast cells and fibroblast cells in a ratio of 50/50 were produced as in Example 1. In the drawings, the portions with light colors correspond to the vascular endothelial cells (HUVEC). Evaluation by allowing human vascular endothelial cells to migrate in each multilayered cell sheet was performed in the same manner as in Example 1. The results are shown in Fig. 14. Investigation of the migration of vascular endothelial cells after culturing for 5 hr revealed that the presence of the fibroblast cells reduced the fluidity in the multilayered myoblast sheet and, as a result, reduced the migration of the vascular endothelial cells. This suggests that in order to actively induce the vascular endothelial cells into the multilayered cell sheet, the fluidity must be enhanced in the multilayered cell sheet. Meanwhile, regarding the formation of a vascular network, a finer network was formed in the multilayered myoblast sheet containing the fibroblast cells. In another investigation, the amount of produced VEGF and the amount of produced HGF in a multilayered cell sheet composed of myoblast sheets only were compared with those in a multilayered cell sheet including myoblast cells and fibroblast cells in a ratio of 50/50. In the multilayered cell sheet composed of myoblast sheets only, the amount of produced VEGF was large and the amount of produced HGF was small compared with those of the multilayered cell sheet including myoblast cells and fibroblast cells in a ratio of 50/50. This suggests that the myoblast cells produced VEGF whereas the fibroblast cells produced HGF. Accordingly, in order to sufficiently develop a vascular network by invaded vascular endothelial cells, a reduction in fluidity in the multilayered cell sheet and the presence of VEGF and HGF are necessary.

### EXAMPLE 3

### Influence of culture state of myoblast cells on cytokine production characteristics

Next, whether the culture state of myoblast cells affects the amount of produced VEGF was investigated. Since the stratified cell sheet is composed of confluent monolayer sheets, the stratified sheet is also three-dimensionally dense, i.e., confluent three-dimensionally, and the cells are in the state of temporarily losing the proliferative properties due to the contact inhibition. Accordingly, the cytokine characteristics in the state in which cells are in dense contact with one another so as to be recognized as a sheet-mimic-system and in the state in which cells can relatively freely migrate were compared. Myoblast cells were seeded at a high density (2.3 × 10⁵ cells/cm²), which was the same as that of the sheet, and at a low density (1.0 × 10⁴ cells/cm²) and were cultured for 48 hr to obtain culture media. The amount of produced VEGF was measured by ELISA. The number of cells when the medium was harvested was counted, and the amount of produced VEGF per cell was determined.

### Experimental conditions

Cell: human skeletal myoblast cell (Np5)
Medium: DMEM (10% FBS)
Culture surface: polystyrene 8-well culture dish, lamin-coated-polystyrene culture surface (myoblast cells proliferation)
Culture environment: 37°C, 5% CO₂ in air
Seeding density: 2.3 × 10⁵ cells/cm² (high density), 1.0 × 10⁵ cells/cm² (low density)
Culture period: 48 h, 168 h
Medium volume: 2.1 mL (medium depth: 0.2 cm)
Medium replacement: each 24 hr
Target: vascular endothelial growth factor (VEGF)
Harvested sample: culture medium at each 24 hr (ELISA)
Number of cells: counted by trypan blue staining

### Results

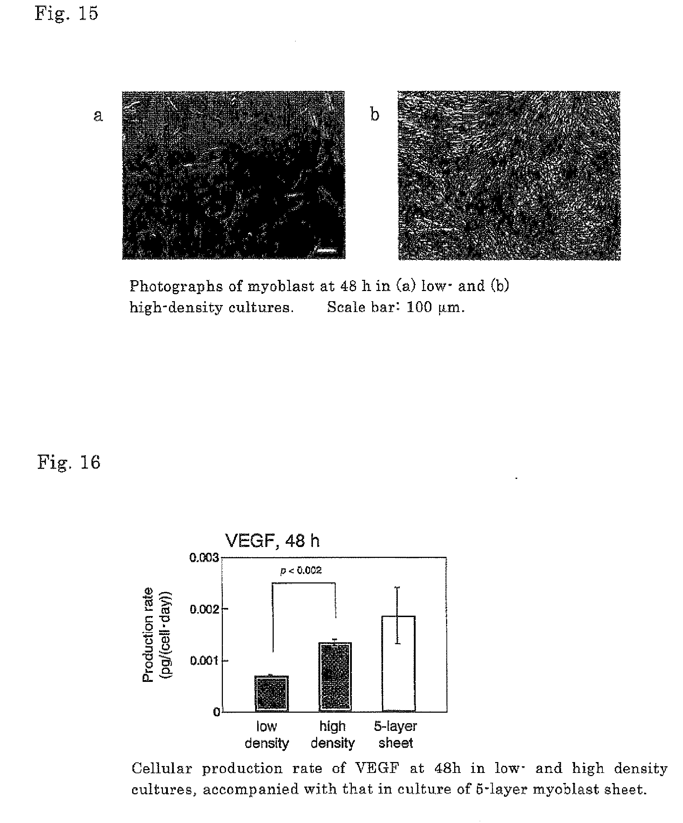

Fig. 15 shows the results after low-density and high-density culture for 48 hr. The cell densities counted by trypan blue staining were (1.22±0.10) × 10⁴ cells/cm² and (1.89±0.14) × 10⁵ cells/cm². Fig. 16 shows the amount of produced VEGF per cell determined from the medium harvested at a culture period of 48 hr, which was calculated by determining the total amount of produced VEGF from the concentration measured by ELISA and a medium volume of 2.1 mL, and then normalizing the total amount by the total number of cells obtained from the cell density and a culture plate area of 10.5 cm². The amount of VEGF produced in the five-layer myoblast sheet at a culture period of 48 hr was collectively shown as the value normalized by the number of cells. The amount per cell in the high density culture was about twice that in the low density culture. This suggests that the VEGF-producing ability of the myoblast cells was increased by generating a dense confluent state. The value in the high density culture was similar to that in the five-sheet culture, and it is believed that it is important to produce a sheet serving as a transplantation material by confluent cells only from the viewpoint of increasing the VEGF secretion ability (secretion efficiency).

### EXAMPLE 4

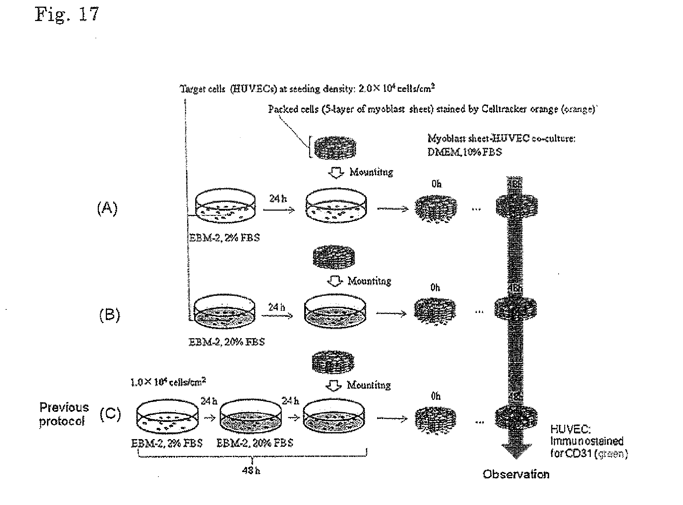

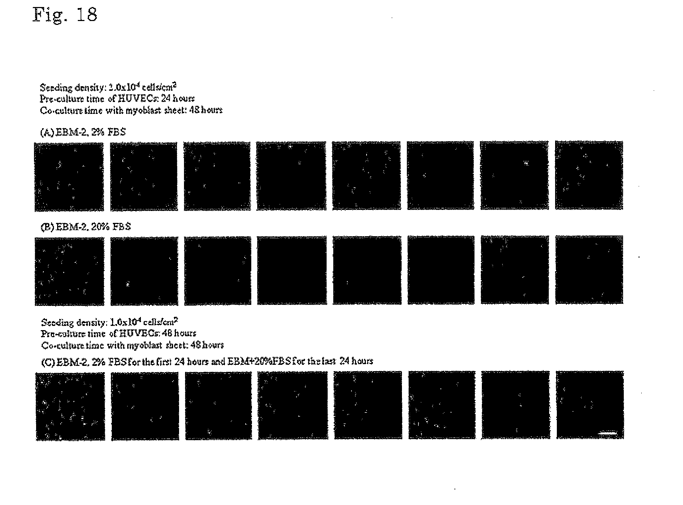

The behaviors of vascular endothelial cells in multilayered myoblast sheets were investigated as in Example 1 except that the vascular endothelial cells were cultured in an EBM-2 medium (containing 2% FBS) for only 24 hr (Fig. 17(A)) or in an EBM-2 medium (containing 20% FBS) for only 24 hr (Fig. 17(B)), instead of the method of Example 1 where the vascular endothelial cells were cultured in an EBM-2 medium (containing 2% FBS) for 24 hr and then in an EBM-2 medium (containing 20% FBS) for 24 hr (Previous protocol in Fig. 17(C)). Fig. 18 shows the appearances of the vascular endothelial cells under conditions of each of (A) to (C) (photographs of randomly selected eight positions in each culture). In the drawings, the portions with light colors correspond to the vascular endothelial cells (HUVEC). The results revealed that the culture conditions (B) form a fine network as in culture conditions (C) shown in Example 1.

### EXAMPLE 5

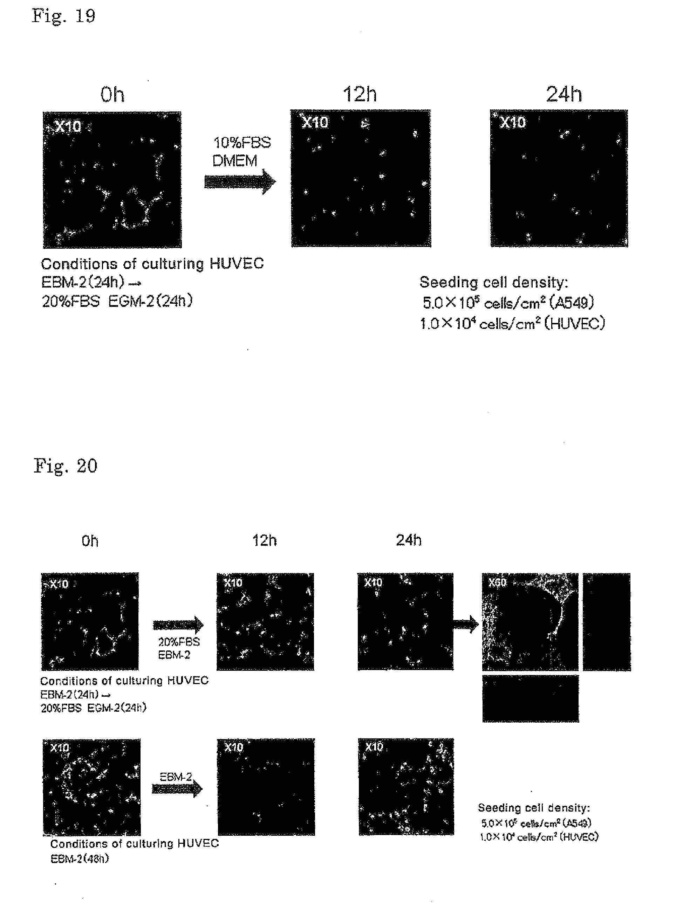

A co-culture system of cancer cells and vascular endothelial cells was constructed. Co-culture of multilayered cancer cell sheet and vascular endothelial cells
A multilayer cancer cell sheet was produced in accordance with the method of Example 1 using an A549 cancer cell line as the cancer cells, wherein 5.0 × 10⁵ cells/cm² of the cells were seeded and cultured in DMEM containing 10% FBS for 24 hr, and then five layers were piled up. Separately, vascular endothelial cells (HUVEC) were cultured (seeding density: 1.0 × 10⁴ cells/cm²) in an EBM-2 medium for 24 hr and then in an EBM-2 medium (containing 20% FBS) for 24 hr. The multilayer cancer cell sheet was overlaid for adhesion onto this HUVEC, and the behaviors of the HUVEC under the A549 multilayered cell sheet at culture periods of 0, 12, and 24 hr were investigated using a confocal microscope as in Example 1. In order to confirm the collapse of a network and the migration of HUVEC in the events from the formation of the network to the migration of HUVEC to the outside of the sheet structure, which were not observed in the previous investigation, observation points (5 hr after the start of co-culture) were added between immediately after the start of the co-culture and 24 hr after the co-culture. Fig. 19 shows the behaviors of HUVEC in the A549 multilayered cell sheet at culture periods of 0, 5, and 24 hr. In the drawings, the portions with light colors correspond to the vascular endothelial cells (HUVEC). It was confirmed that the formation of the network by the HUVEC under the A549 multilayered cell sheet already progressed even at immediately after the transfer of the cell sheet (0 hr). At this point of time, the migration of HUVEC to the outside of the sheet-like cell clusters did not seem to progress. The appearance after 5 hr showed that the collapse of the network and the migration of HUVEC to the outside of the multilayered cell sheet already started and that HUVEC started to be noticeably present near the outer boundaries of the sheet-like cell clusters. After that, the collapse of the network and the migration of HUVEC progressed, and at the time of 24 hr, HUVEC was hardly observed in the multilayered cell sheet. The results at 48 hr were approximately the same. On the outside of the multilayer cancer cell sheet, a large number of HUVEC were observed. The migration of the vascular endothelial cells to the outside of the multilayered cell sheet was also observed in the case of using myoblast cells as the packed cells, but in the case of using A549 cells as the packed cells, the formation of the network, its collapse, and the migration of vascular endothelial cells to the outside of the multilayered cell sheet progressed very rapidly.

### EXAMPLE 6

A co-culture system of cancer cells and vascular endothelial cells was constructed under conditions different from those of Example 5.

### Co-culture of multilayered cancer cell sheet and vascular endothelial cells

As shown in Fig. 20, HUVEC was co-cultured with the A549 multilayered cell sheet as in Example 5 except that the medium after the adhesion of the multilayer cancer cell sheet to the HUVEC was an EBM-2 medium (containing 2% FBS), which was a dedicated medium for HUVEC, (photographs at the bottom stage in Fig. 20) or an EBM-2 medium (containing 20% FBS) (photograph at the central stage in Fig. 20), instead of the DMEM containing 10% FBS (photographs at the top stage in Fig. 20) in Example 5. In the drawings, the portions with light colors correspond to the vascular endothelial cells (HUVEC). The results revealed that in both the EBM-2 medium (containing 2% FBS) and the EBM-2 medium (containing 20% FBS), aggregation of the HUVEC was maintained by continuing the co-culture with the A549 multilayered cell sheet.

### EXAMPLE 7

A co-culture system of mesenchymal stem cells and vascular endothelial cells was constructed.

### Co-culture of multilayered mesenchymal stem cell sheet and vascular endothelial cells

A multilayer sheet of mesenchymal stem cells was produced in accordance with the method in Example 1 using adipose-derived mesenchymal stem cells, wherein 4.5 × 10⁵ cells/cm² of the cells were seeded and cultured in an α-MEM containing 10% FBS for 48 hr, and then five sheets were layered. Separately, vascular endothelial cells (HUVEC) were cultured (seeding density: 1.0 × 10⁴ cells/cm²) in an EBM-2 medium for 24 hr and then in an EBM-2 medium (containing 20% FBS) for 24 hr. The multilayered mesenchymal stem cell sheet was overlaid for adhesion onto this HUVEC, and the behaviors of the HUVEC under the multilayered mesenchymal stem cell sheet were investigated using a fluorescence microscope as in Example 1. The results revealed that the HUVEC under the multilayered mesenchymal stem cell sheet moved to the uppermost layer of the multilayered mesenchymal stem cell sheet at a culture period of 48 hr.

### EXAMPLE 8

A co-culture system ofmyocardial cells and vascular endothelial cells was constructed.

### Co-culture of multilayered myocardial cell sheet and vascular endothelial cells

A multilayer sheet of myocardial cells was produced in accordance with the method in Example 1 using rat myocardial cells, wherein 5.5 × 10⁵ cells/cm² of the cells were seeded and cultured in an M199 medium containing 10% FBS for 24 hr, and then five sheets were layered. Separately, vascular endothelial cells (HUVEC) were cultured (seeding density: 1.0 × 10⁴ cells/cm²) in an EBM-2 medium for 24 hr and then in an EBM-2 medium (containing 20% FBS) for 24 hr. The multilayered myocardial cell sheet was overlaid for adhesion onto this HUVEC, and the behaviors of the HUVEC under the multilayered myocardial cell sheet were investigated using an optical microscope as in Example 1. The results revealed that the HUVEC under the multilayered myocardial cell sheet moved to the uppermost layer of the multilayered myocardial cell sheet at a culture period of 96 hr.

### EXAMPLE 9

A co-culture system of epidermal ketatinocyte cells and fibroblast cells was constructed.

### Co-culture of multilayered epidermal ketatinocyte cell sheet and fibroblast cells

A stratified sheet of epidermal ketatinocyte cells was produced using epidermal ketatinocyte cells by a common method using 3T3 feeder cells, wherein 4.5 × 10⁴ cells/cm² of the cells were seeded and cultured in a DMEM medium containing 10% FBS for 14 days to obtain a stratified epidermal ketatinocyte cell sheet with about three layers. Aside from this, fibroblast cells were cultured (seeding density: 1.0 × 10⁴ cells/cm²) in a DMEM medium for 24 hr. The stratified epidermal ketatinocyte cell sheet was overlaid for adhesion onto this fibroblast cells, and the behaviors of the fibroblast cells under the stratified epidermal ketatinocyte cell sheet were investigated using a fluorescence microscope as in Example 1. The results revealed that most of the fibroblast cells under the stratified epidermal ketatinocyte sheet did not move into the stratified epidermal ketatinocyte cell sheet even at the culture period of 48 hr.

### INDUSTRIAL APPLICABILITY

In the cell evaluation system according to the present invention, it is possible to obtain three-dimensional information on a biological parameter relating to at least one selected from viability, proliferating ability, migration, and differentiation of cultured cells by a simple two-dimensional analytical technique. The cell evaluation system of the present invention does not need an expensive, large-scale analyzer, which has conventionally needed, and can be easily combined with any peripheral cell culture apparatus to obtain refined information. Accordingly, the present invention is significantly useful in the fields of, for example, drug discovery, pharmaceutics, medicine, and biology.

### REFERENCE SIGNS LIST

Reference signs in the drawings have the following meanings.

N_{G}: the number of green voxels in one image slice [voxel]
N_{R}: the number of red voxels in one image slice [voxel]
R_{G}: the ratio of the green voxels in one image slice [-]
h: the height of photographed image slice [µm]
N_{G},max: the maximum number of the green voxels in image slices [-]
N_{R},max: the maximum number of the red voxels in image slices [-]
N_{G}/N_{G,}max: the ratio of the number of green voxels to the maximum number in each slice [-]
N_{R}/N_{R},max: the ratio of the number of red voxels to the maximum number in each slice [-] z: sheet thickness [µm]
F_{G}: the frequency of green voxels in each layer [-]
1: the total length of a vascular network in one image [mm/image]
t: the number of tips per 1 mm of a vascular network [tip/mm]

## Claims

1. A cell evaluation system comprising a multilayered cell sheet, a target cell, and a two-dimensional analyzer for obtaining three-dimensional information on a biological parameter relating to at least one selected from viability, proliferating ability, migration, and differentiation of the target cell by a two-dimensional analytical technique.

2. The cell evaluation system according to Claim 1, wherein the system is loaded with a drug.

3. The cell evaluation system according to Claim 1 or 2, wherein the multilayered cell sheet comprises layered cell sheets produced on a temperature-responsive culture plate coated with a temperature-responsive polymer having a phase transition temperature of 0 to 80°C.

4. The cell evaluation system according to any one of Claims 1 to 3, wherein the multilayered cell sheet is piled up by three or more monolayered cell sheets.

5. The cell evaluation system according to any one of Claims 1 to 4, wherein a cell constituting the multilayered cell sheet flows in the multilayered cell sheet.

6. The cell evaluation system according to Claim 5, wherein the cell constituting the multilayered cell sheet comprises at least one cell selected from a myoblast cell, a myocardial cell, and a vascular endothelial cell.

7. The cell evaluation system according to any one of Claims 1 to 4, wherein the cell constituting the multilayered cell sheet does not flow in the multilayered cell sheet.

8. The cell evaluation system according to Claim 7, wherein the cell constituting the multilayered cell sheet comprises a fibroblast cell or an epidermal ketatinocyte cell.

9. The cell evaluation system according to any one of Claims 1 to 4, wherein the cell constituting the multilayered cell sheet shows regulated fluidity in the multilayered cell sheet.

10. The cell evaluation system according to any one of Claims 1 to 9, wherein the target cell is a fluorescence-labeled cell.

11. The cell evaluation system according to Claim 10, wherein the two-dimensional analytical technique is a fluorescence microscope.

12. A method of evaluating a cell, comprising obtaining three-dimensional information on a biological parameter relating to at least one selected from viability, proliferating ability, migration, and differentiation of the target cell by a two-dimensional analytical technique using the cell evaluation system according to any one of Claims 1 to 11.

13. The method of evaluating a cell according to Claim 12, wherein the biological parameter of the target cell is traced by adding a drug to the system.

14. A method of evaluating a mechanism for constituting a vascular network in tissue, comprising tracing migration behavior of the target cell in the cell evaluation system with a fluorescence microscope, wherein the cell constituting the multilayered cell sheet is a myoblast cell; and the labeled cell is a fluorescence-labeled vascular endothelial cell.

15. A method of evaluating a drug efficacy of an anticancer agent, comprising using the cell evaluation system, wherein the cell constituting the multilayered cell sheet is a cancer cell; the labeled cell is a fluorescence-labeled vascular endothelial cell; and the drug is an anticancer agent.

16. A method of evaluating a stem cell differentiation-programming mechanism, comprising using the cell evaluation system, wherein the cell constituting the multilayered cell sheet is a differentiated cell; the labeled cell is a fluorescence-labeled stem cell; and the drug is a differentiation-inducing agent.
